# EUROPEAN PATENT APPLICATION

(11) **EP 4 814 977 A1**
(43) Date of publication of application: **30.09.2026**
(21) Application number: 26163012.3
(22) Date of filing: 06.03.2026
(51) Int. Cl.: G16H 30/40, G16H 50/50

(54) **SYSTEM AND METHOD FOR GENERATING A THREE-DIMENSIONAL DIGITAL PATIENT MODEL OF A PATIENT'S ORAL ANATOMY HAVING AN EDENTULOUS JAW PORTION**

(30) Priority: 24.03.2025 DE 102025111357
(71) Applicant: Institut Straumann AG, 4002 Basel (CH)
(72) Inventor: Marbach, Julien, Montreal, QC (CA); Svensson, Leif, Basel (CH)
(74) Representative: Fleuchaus & Gallo Partnerschaft mbB

(57) **Abstract**

Computer-implemented method, computer program product and computing system (100) for generating a three-dimensional digital patient model (210) of a patient's oral anatomy with the patient (1) having a first at least semi-edentulous jaw arch, and having at least one first dental implant placed in the jawbone of said first at least semi-edentulous jaw arch, and having a prosthesis applied to the at least semi-edentulous jaw arch. The system receives a second three-dimensional intra-oral surface scan dataset (202) obtained from the patient's jaw arch opposing the at first least semi-edentulous jaw arch in a non-occlusion state and a third dataset (203) comprising representations of both jaw arches in an occlusion state. A fourth three-dimensional intra-oral surface dataset (204) of at least portions of the gingiva surface (204-g) in said first at least semi-edentulous jaw arch and with surface information of a first implant head interface (204-1, 204-2, 204-3, 204-4) of the at least one first implant located in the at least semi-edentulous jaw arch is received. A fifth three-dimensional 4π surface dataset (205) of the prosthesis is received with surface information of at least one first abutment interface (205-1, 205-2, 205-3, 205-4) configured to engage positively with the implant head interface (204-1, 204-2, 204-3, 204-4). The digital patient aligns the fourth three-dimensional intra-oral surface dataset (204) and the fifth three-dimensional 4π surface dataset (205) with the second three-dimensional intra-oral surface scan dataset (202) and with the third three-dimensional intra-oral surface scan dataset (203).

## Description

### Technical Field

The present disclosure in general relates to the generation of a digital twin, and in particular relates to generating a digital patient model of a patient's oral anatomy having an at least semi-edentulous jaw portion with a prosthesis applied thereto.

### Background

A portion of a jaw arch of a human patient which has no teeth in at least one half of the upper or lower jaw is referred to as a semi-edentulous jaw arch herein. A jaw arch, which has no teeth at all, is referred to as fully-edentulous jaw arch. A patient may have one or more semi-edentulous jaw arches (up to four in case of having no teeth at all). An at least semi-edentulous jaw arch per the present disclosure refers to a semi-edentulous our fully-edentulous jaw arch herein.

Typically, a prosthesis is applied to such edentulous jaw portions. The fabrication of such a prosthesis is critical in that it must fit the patient's edentulous jaw portions very precisely and in such way that the upper and lower jaws properly engage and occlude with each other to avoid inconvenience in the patient's mastication, and to keep the patient's natural facial impression.

Traditionally, fabrication of a prosthesis typically involves making a mold of the patient's edentulous jaw portion using a resin material to obtain a preliminary impression. A plaster model is made using the preliminary impression to make a master cast for the edentulous jaw portion. A recording base is then made on the master cast by using resin or wax, and a wax rim which is placed on the recording base. Artificial teeth are arranged in an appropriate position, direction, and height on such a wax rim. At this time, the position and direction of the artificial teeth, which are to be installed on the prosthesis, are adjusted by using a device such as an articulator in consideration of the occlusion relationship.

In this process, a wax rim-type prosthesis is fitted to the edentulous jaw of the patient several times to check and adjust the occlusion state. The process of arranging artificial teeth on a wax rim is performed mostly by hand, so the degree of completion is greatly influenced by the experience and skill of an operator.

In addition, in the process of fabricating a wax rim prosthesis, the patient has to visit the dentist several times, and the operator also has to adjust the occlusal vertical dimension and the arrangement of the artificial teeth on the wax-rim several times, imposing quite some burden on the patient and the operator. When the wax rim prosthesis fabrication is completed, the wax rim prosthesis is placed in a mold, the wax rim is melted and removed with hot water, and then a resin replacing the wax rim is filled and cured, thereby completing the prosthesis fabrication.

Typically, such a prosthesis adheres to the edentulous portion of the jaw merely through its form-fitting shape. However, there can be disadvantages of such a prosthesis with regard to the strength and durability of the material and with regard to the stability of the connection between the prosthesis and the edentulous jaw portion. Additionally, the requirements for precision are very high to avoid stress to the patient's soft or hard tissue which can have severe consequences for the patient, such as tissue damage or pain.

Therefore, it may be desirable to provide a prosthesis which can be made of a more permanent and rigid material, and which is firmly attached to the patient's jaw via respective implants in the patient's edentulous jaw portion. A prosthesis rigidly mounted on one or more jaw implants must, however, also be very precisely manufactured and must not cause any tension or stress on the implant in the patient's jawbone as such tension or stress can, again, lead to damage to the bone tissue of the patient.

### Summary

There is therefore a desire to support a highly accurate design of a prosthesis applied to an at least semi-edentulous jaw portion of a patient, which is firmly connected with the patient's jaw via one or more respective dental implants. Such support is provided by a highly accurate digital patient model of the patient's oral anatomy which is generated by a computer-implemented method executed by a respective computer system as disclosed herein.

The generated digital patient model including said prosthesis, which may be a traditional preliminary prosthesis or a former prosthesis requiring replacement, ensures an accurate match of a prosthesis designed and manufactured using the digital patient model of the patient's oral anatomy generated by a computer-implemented method of the present disclosure. The method of the present disclosure obtains a correction relative position of the at least semi-edentulous jaw portion(s) also in an occlusion state-while the patient wears an existing prothesis, and places the models of such prostheses in position in the digital patient model in such way that no tension is exerted on the implant(s) and thus the jaw bone by the prosthesis if the prosthesis is fabricated in accordance with the generated digital patient model of the patient's oral anatomy.

In a first aspect, a computer-implemented method running in a computer system is provided for generating a three-dimensional digital patient model of a patient's oral anatomy. In other words, the method generates a digital twin of the patient's oral anatomy. The patient has a first at least semi-edentulous jaw arch and has at least one first dental implant placed in the jawbone of said first at least semi-edentulous jaw arch. The generated three-dimensional digital patient model provides an accurate representation of the position and orientation of said implant in the patient's jawbone as well as the first at least semi-edentulous jaw arch relative to the opposing jaw of the patient. The method is executed by one or more processors of a computer system and comprises steps which are disclosed in the following.

The computer system, in a step of the method, receives second and third three-dimensional intra-oral surface scan datasets obtained from the patient's oral anatomy with the semi-edentulous jaw arch while having a first prosthesis applied to the first at least semi-edentulous jaw arch. In the field of digital dental technology, scan datasets of anatomical structures are generally obtained optically or radiologically. Optical surface scan data is typically preferred for the evaluation or computation of virtual tooth restorations due to the ability to achieve high precision without exposing the patient to high-level dose of radiation.

Optical (intra-oral) scanners for three-dimensional measurement directly from intra-oral surface structures, or from extra-oral impressions of oral surface structures, are widespread. Usually, surface data is represented by a surface mesh comprising triangular elements, which are routinely saved and exchanged between systems in STL or similar surface definition formats. The computer system has a respective data interface configured to receive datasets in such formats.

The second three-dimensional intra-oral surface scan dataset comprises a digital three-dimensional surface representation of at least a portion of the patient's jaw arch located opposite the first at least semi-edentulous jaw arch. For example, if the patient has no teeth in one half of the upper jaw arch, the first prosthesis is applied to this edentulous portion of the upper jaw semi-edentulous jaw arch. The second intra-oral surface scan dataset may then include the teeth of the opposing half of the lower jaw arch and adjacent soft tissue of the lower jaw.

It is also possible that the lower jaw arch has an edentulous portion, too, with a respective second prosthesis. An alternative aspect, described further down below, is adapted to generate the digital patient model also for a patient having at least semi-edentulous jaw arches in the upper and lower jaws.

Optionally, the system may, in a further step, also receive a first three-dimensional intra-oral surface scan dataset that comprises a digital three-dimensional surface representation of the first at least semi-edentulous jaw arch. The first intra-oral surface scan dataset is obtained while the prosthesis is applied to the semi-edentulous jaw arch and also includes a three-dimensional surface representation of the visible parts of the prosthesis and adjacent soft tissue of the upper jaw. In case the patient has a fully-edentulous jaw arch, the first prosthesis may apply to the entire jaw arch.

The third three-dimensional intra-oral surface scan dataset comprises a digital three-dimensional surface representation of at least a part of at least one of the patient's posterior buccal or lingual jaw portions while in an occlusion state. The third intra-oral surface scan dataset is sometimes referred to as bite scan. The bite scan shows the position and orientation of the opposing upper and lower jaw portions with the prosthesis being applied and can be seen as a reference configuration of the patient's oral anatomy in the occlusion state which needs to me matched by any prosthesis to be designed and/or manufactured in accordance with the digital patient model generated according to the present disclosure. In practice, it is not always necessary to obtain the third intra-oral surface scan dataset for the full upper and lower jaw arches. Rather, representations of the patient's posterior buccal or lingual opposing jaw portions are sufficient for serving as reference configuration of the oral anatomy.

The system, in another step of the method, further receives a fourth three-dimensional intra-oral surface dataset of at least portions of the gingiva surface of said first at least semi-edentulous jaw arch of the patient's oral anatomy. The fourth three-dimensional intra-oral surface dataset comprises surface information of a first implant head interface of the at least one first implant located in the first at least semi-edentulous jaw arch. The first implant head interface is exposed through the gingiva.

In other words, the fourth intra-oral surface dataset includes a representation of the surface of the semi-edentulous jaw arch which is hidden by the first prosthesis when being applied. The at least one first implant is used for positioning and affixation of the first prosthesis on the first at least edentulous jaw arch. For this purpose, the first prosthesis has at least one first abutment interface configured to engage substantially positively with the first implant head interface of the at least one first implant. Alternative ways for obtaining the fourth intra-oral surface dataset are described in detail further down below.

The system, in a further step, receives a fifth three-dimensional 4π surface dataset comprising a three-dimensional surface representation of the first prosthesis. A 4π surface dataset as used herein is a data set which includes data that could be obtained by a full 4π measurement, i.e., a measurement from at least sufficiently many but most of the time substantially any direction of a surrounding sphere (a sphere surrounding the first prosthesis).

In other words, the fifth 4π surface dataset includes surface information of the prosthesis from sufficiently many or substantially all viewing angles of the surrounding sphere of the prosthesis. Alternative ways for obtaining the fifth 4π surface dataset are described in detail further down below. The fifth surface dataset comprises a three-dimensional surface representation of at least one first abutment interface configured to engage positively with the first implant head interface of the at least one first implant located in the first at least semi-edentulous jaw arch when the first prosthesis is applied.

The system has an alignment module which, in a further step of the method, is adapted to align the fourth three-dimensional intra-oral surface dataset and the fifth three-dimensional 4π surface dataset of the first prosthesis relative to each other in a common reference frame by registering the surface information of the first implant head interface of the at least one first implant with the surface information of the at least one first abutment interface. This alignment step ensures that the first prosthesis in the digital patient model is accurately positioned with regard to the semi-edentulous jaw portion of the patient such that any abutment interface of the first prosthesis substantially matches the corresponding implant head interface.

"Substantially matching" or "substantially positively fitting", or "aligning" in the context of the present disclosure means that surfaces, such as, for example, an abutment interface and its corresponding implant head interface, have a position and orientation relative to one another in the digital patient model which represents a digital representation of a substantially positive fit of the two surfaces. For example, when the first prosthesis is applied to the edentulous jaw arch portion such that, in the real world, no forces would be exerted by the abutment interface(s) on the corresponding implant head interface(s), the digital model represents this as a substantially positive fit between surfaces or, simply spoken, aligned surfaces. To achieve such a position and orientation of two surfaces, or their digital representation, various methods can be applied, such as, for example, an automated method of finding the minimal sum of the square of the distances of vertexes of the surface mesh representations of the respective surfaces, or a manual positioning.

The alignment module is, in a further step of the method, adapted to align the fifth three-dimensional 4π surface dataset with the second three-dimensional intra-oral surface scan dataset relative to each other in a common reference frame by registering portions of the fifth three-dimensional 4π surface dataset and portions of the second three-dimensional intra-oral surface scan dataset with the third three-dimensional intra-oral surface scan dataset. The alignment of the fourth three-dimensional intra-oral surface dataset and the fifth three-dimensional 4π surface dataset may be maintained so that the second alignment step may also ensure that the previously aligned fourth intra-oral surface dataset and the fifth 4π surface dataset are also aligned with the second intra-oral surface scan dataset of the opposing jaw arch so that the entire oral anatomy of the patient is represented accurately and with high precision.

In an alternative second aspect, a modification of the above-described method and system may be applied in case the patient has a second at least semi-edentulous jaw arch in the jaw located opposite the first at least semi-edentulous jaw arch. In this case, the step of receiving of the second three-dimensional intra-oral surface scan dataset comprising a digital three-dimensional surface representation of the second at least semi-edentulous jaw arch while having also a second prosthesis applied to the second at least semi-edentulous jaw arch is optional. Processing steps similar to those of the method performed in the first embodiment with regard to the first at least semi-edentulous jaw arch are also performed with regard to the second at least semi-edentulous jaw arch in this aspect.

In this second aspect, in a first optional step, the system receives a third three-dimensional intra-oral surface scan dataset obtained from the patient's oral anatomy, including the first at least semi-edentulous jaw arch while having a first prosthesis applied to the first at least semi-edentulous jaw arch and a second prosthesis applied to the second at least semi-edentulous jaw arch. In the second aspect, the third three-dimensional intra-oral surface scan dataset comprises a digital three-dimensional surface representation of at least a part of the patient's posterior buccal or lingual jaw portions while in an occlusion state.

Similar to the first aspect, in a further step, the system receives, a fourth three-dimensional intra-oral surface dataset of at least portions of the gingiva surface of said first at least semi-edentulous jaw arch of the patient's oral anatomy. The fourth three-dimensional intra-oral surface dataset comprises surface information of a first implant head interface of the at least one first implant located in the first at least semi-edentulous jaw arch, the first implant head interface being exposed through the gingiva.

Then, again similar to the first aspect, the system receives, in a further step, a fifth three-dimensional 4π surface dataset comprising a three-dimensional surface representation of the first prosthesis as well as surface information of at least one first abutment interface configured to engage positively with the first implant head interface of the at least one first implant located in the first at least semi-edentulous jaw arch when the first prosthesis is applied.

In the second aspect, in a further step, the system additionally receives a sixth three-dimensional intra-oral surface dataset of at least portions of the gingiva surface of said second at least semi-edentulous jaw arch of the patient's oral anatomy. The sixth three-dimensional intra-oral surface dataset comprises surface information of a second implant head interface of the at least one second implant located in the second at least semi-edentulous jaw arch, the second implant head interface being exposed through the gingiva.

In a further step, the system receives a seventh three-dimensional 4π surface dataset comprising a three-dimensional surface representation of the second prosthesis as well as surface information of at least one second abutment interface configured to engage positively with the second implant head interface of the at least one second implant located in the second at least semi-edentulous jaw arch when the second prosthesis is applied.

The fourth and sixth three-dimensional intra-oral surface datasets as well as the fifth and seventh three-dimensional intra-oral surface datasets are respectively similar in nature, however, may refer to portions of at least both jaw archs of the patient.

Similar to the first aspect, in a further step, the an alignment module of the system aligns the fourth three-dimensional intra-oral surface dataset and the fifth three-dimensional 4π surface dataset of the first prosthesis relative to each other in a common reference frame by registering the surface information of the first implant head interface of the at least one first implant with the surface information of the at least one first abutment interface.

In addition, in a further step of the second aspect, the alignment module of the system further aligns the sixth three-dimensional intra-oral surface dataset and the seventh three-dimensional 4π surface dataset of the second prosthesis relative to each other in a common reference frame by registering the surface information of the second implant head interface of the at least one second implant with the surface information of the at least one second abutment interface.

In a further step, the alignment module of the system aligns the fifth three-dimensional 4π surface dataset with the seventh three-dimensional 4π surface scan dataset relative to each other in the common reference frame by registering portions of the fifth three-dimensional 4π surface dataset and portions of the seventh three-dimensional 4π surface scan dataset with respective portions of the third three-dimensional intra-oral surface scan dataset. The alignment of the fourth three-dimensional intra-oral surface dataset and the fifth three-dimensional 4π surface dataset as well as the sixth three-dimensional intra-oral surface dataset and the seventh three-dimensional 4π surface dataset may be maintained so that the third alignment step may also ensure that the previously aligned fourth intra-oral surface dataset and the fifth 4π surface dataset are also aligned with the previously aligned sixth intra-oral surface dataset and the seventh 4π surface dataset of the opposing jaw arch so that the entire oral anatomy of the patient is represented accurately and with high precision.

In an embodiment, the position and orientation of a particular first implant head interface can be derived from an eighth three-dimensional intra-oral surface scan dataset obtained from a corresponding first at least semi-edentulous jaw arch while having the respective first prosthesis removed. With the prosthesis being removed, a so-called scan-body is mounted on the respective first implant(s).

A scan-body is known by a skilled person as a special digital positioning device used in high-precision scanning technology to accurately capture the three-dimensional structural data of a patient's oral cavity. The scan-body has a known geometry and its position and orientation in relation to said first at least semi-edentulous jaw arch can be determined from the eighth intra-oral surface scan dataset. This allows to compute, with high accuracy, the position and orientation of the respective first implant head interface to which the scan-body is attached and thus also the implant position and orientation.

Generally, the geometry of the respective implant (including the implant head interface) may be known. For example, the geometry of the implant may be known from an existing CAD model of the implant, or from a technical specification of the implant. The geometry of the implant may also be determined from radiological data previously obtained by radiological measurements (e.g., a volumetric CT scan or X-ray images). The computation of the position and orientation of the implant head interface is based on position, orientation and the known geometry of the scan-body, as well as the known geometry of the implant to which the scan-body is affixed. The result of the computation of said implant head interface position and orientation has an accuracy which is high enough to allow fixation of the prosthesis to the respective implant head via a corresponding abutment interface without exerting any stress or tension on the patient's soft or hard tissue, such as, the gingiva or the jaw bone.

In case the patient has two at least semi-edentulous jaws, a nineth three-dimensional intra-oral surface scan dataset may be obtained from the second at least semi-edentulous jaw arch opposite the first at least semi-edentulous jaw arch, that is, opposite of the eighth three-dimensional intra-oral surface scan dataset with the second prosthesis removed and scan-bodies attached to particular second implants. Then, the position and orientation of respective second implants and their second implant head interfaces can be determined in the same manner by using respective scan-bodies also for the second implants of the second at least semi-edentulous jaw arch.

In one embodiment, the fourth three-dimensional intra-oral surface dataset and/or the sixth three-dimensional intra-oral surface dataset is derived. For example, by computation in the digital domain, from the eighth three-dimensional intra-oral surface scan dataset and/or nineth three-dimensional intra-oral surface scan dataset, respectively. This may be achieved by detecting at least one scan-body position of at least a respective scan-body, and cleaning, from the eighth three-dimensional intra-oral surface scan data set, at least one modification area around the at least one detected scan-body position.

A modification area, as used herein, denotes a portion of the eighth or nineth three-dimensional intra-oral surface scan dataset which comprises a representation of a scan-body. Cleaning the modification area includes the removal of the scan-body representation from the respective modification area. Then, the at least one cleaned modification area is supplemented with the surface information of the known geometry of the respective implant head interface in accordance with the known geometry and the computed position and orientation of the respective scan-body within the eighth or nineth three-dimensional intra-oral surface scan dataset.

Various alternative methods for cleaning the at least one modification area may be used.

For example, in a first alternative, a clone copy is generated from the eighth surface scan dataset with a data modification tool adapted to remove each scan-body representation from the eighth three-dimensional intra-oral surface scan data set. For example, an appropriate brush and/or lasso tool adapted for use with three-dimensional surface areas as data modification tool may be used in a system employing a graphical user interface. Such a brush tool allows to select surface areas of the three-dimensional intra-oral surface dataset, and, in particular, its mesh representation, using an additive "paint-on" method, with a variable sized brush. Similarly, a lasso tool allows to select surface areas of the three-dimensional intra-oral surface dataset, and, in particular, its mesh representation, by outlining these areas, using a polygonal or a free form "lasso".

A clone copy may be generated as a deep copy of a source dataset. In information technology, a deep copy generally represents a replica of an object that doesn't share the same references as the original. That way, while making changes to the duplicate (clone copy), the original (source dataset) remains unmodified. On the other hand, a shallow copy shares the original's properties and references, so when changing the copy, the original might be modified alongside with it.

For example, in a second alternative, a deep copy or a shallow copy is generated from the eighth and/or nineth three-dimensional intra-oral surface scan dataset and a suitably trained artificial intelligence tool is used to automatically remove each scan-body representation from the respective modification area of the eighth and/or nineth three-dimensional intra-oral surface scan dataset.

Supplementing the at least one cleaned modification area may be achieved by using, for example, any of the following alternatives.

In an exemplary first alternative, a three-dimensional intra oral gingiva surface scan dataset is obtained for the respective cleaned modification area with the respective scan-body being physically removed from the respective implant. The cleaned modification area may then be supplemented with the respective portion of the gingiva surface scan dataset comprising a three-dimensional representation of the respective implant head interface. In this process, the representation of the respective implant head interface may be merged, e.g. added, into the modification area such that it is accurately placed at the position and with the orientation derived from the earlier computation of said implant head interface position and orientation within the respective three-dimensional intra oral surface dataset.

In a second exemplary alternative, the supplementing of the modification area is achieved by a mere computation without a need for any further scan step. For each cleaned modification area, three-dimensional surface information of a respective implant head interface with known geometry is computed based on a known geometry as well as the position and orientation of the respective scan-body.

The purpose of using a scan-body is to determine, with a very high degree of accuracy, the position and orientation of the implant head interface to which the scan-body is mounted. This information is then used to merge the computed implant head interface surface information at the correct position into the cleaned modification area of the respective surface dataset. In this alternative, there is substantially no surface information with regard to the gingiva surrounding the implant head interface being injected into the cleaned modification area.

In embodiments where receiving the third three-dimensional intra-oral surface scan dataset further comprises receiving a first three-dimensional intra-oral surface scan dataset and/or receiving a second three-dimensional intra-oral surface scan dataset, obtained from the patient's oral anatomy, the fifth and/or seventh three-dimensional 4π surface dataset of the first and/or second prosthesis may be obtained by any of the following exemplary alternatives. The first three-dimensional intra-oral surface scan dataset comprises a digital three-dimensional surface representation of at least a portion of the patient's first at least semi-edentulous jaw arch with the first prosthesis applied, and the second three-dimensional intra-oral surface scan dataset comprises a digital three-dimensional surface representation of at least a portion of the patient's second at least semi-edentulous jaw arch with the second prosthesis applied.

In a first exemplary alternative, the first and/or second three-dimensional intra-oral surface scan dataset is used as a basis. A clone copy (e.g., a deep copy) of the representation of the first and/or second prosthesis in the first and/or second three-dimensional intra-oral surface scan dataset, respectively, is generated. In this clone copy, soft tissue representations are removed from the representation of the respective prosthesis. As a result, a first portion of the fifth and /or sevenths three-dimensional 4π surface dataset is obtained.

The complementary second portions of the surface information of the respective prosthesis with at least one respective abutment interface is then obtained from a continued surface scan of the respective prosthesis while being removed from the oral anatomy of the patient. Once the respective prosthesis is removed from the patient's oral cavity, the prosthesis portions being hidden (not visible for the intra oral scanner) in the first and/or second three-dimensional intra-oral surface scan dataset become visible and the continued surface scan generates the complementary second portions of the fifth and /or seventh three-dimensional 4π surface dataset. As a result, a three-dimensional data set is obtained which represents the surface of the respective entire prosthesis including the respective one or more abutment interfaces from substantially all viewing angles.

In a second exemplary alternative, the fifth and/or seventh three-dimensional 4π surface dataset with the surface information of at least one first or second abutment interface is obtained from a full 4π surface scan of the respective prosthesis while being removed from the oral cavity of the patient. The second alternative renounces on reusing the surface information of the respective prosthesis which is already available in the first and/or second intra-oral surface scan dataset.

In a third exemplary alternative, the fifth and/or seventh three-dimensional 4π surface dataset with the surface information of the respective at least one abutment interface is obtained from an existing digital three-dimensional geometric model of the respective prosthesis. Such a geometric model of the respective prosthesis may already exist in cases where the prosthesis was designed using digital design tools, such as a CAD tool.

In one embodiment, a computer program product is provided for generating a three-dimensional digital patient model of a patient's oral anatomy. The computer program product comprises computer-readable instructions that, when loaded into a memory of a computing device and processed by one or more processors or the computing device, cause the computing device to perform any of the steps of the above- disclosed computer-implemented methods. It is noted that the above disclosure refers to steps being implemented or executed by respective computerized systems or sub-modules thereof.

Further aspects and embodiments of the invention will become apparent by means of the elements and combinations particularly depicted in the appended claims. It is to be understood that both the foregoing general description and the following detailed description are exemplary and explanatory only and are not restrictive of the invention as described.

### Brief description of the figures

FIG. 1 is a schematic diagram showing an embodiment a computer system for generating a three-dimensional digital patient model of a patient's oral anatomy according to an embodiment;
FIG. 2 is a flow-chart of a computer-implemented method for generating a three-dimensional digital patient model of a patient's oral anatomy according to an embodiment;
FIG. 3 illustrates elements of the patient's oral anatomy represented by datasets used for generating a digital patient model according to various embodiments;
FIG. 4 illustrates a definition of a three-dimensional 4π surface dataset example;
FIG. 5 is a diagram showing an example of a generic computer device and a generic mobile computer device, which may be used with the techniques described herein.

### Detailed description

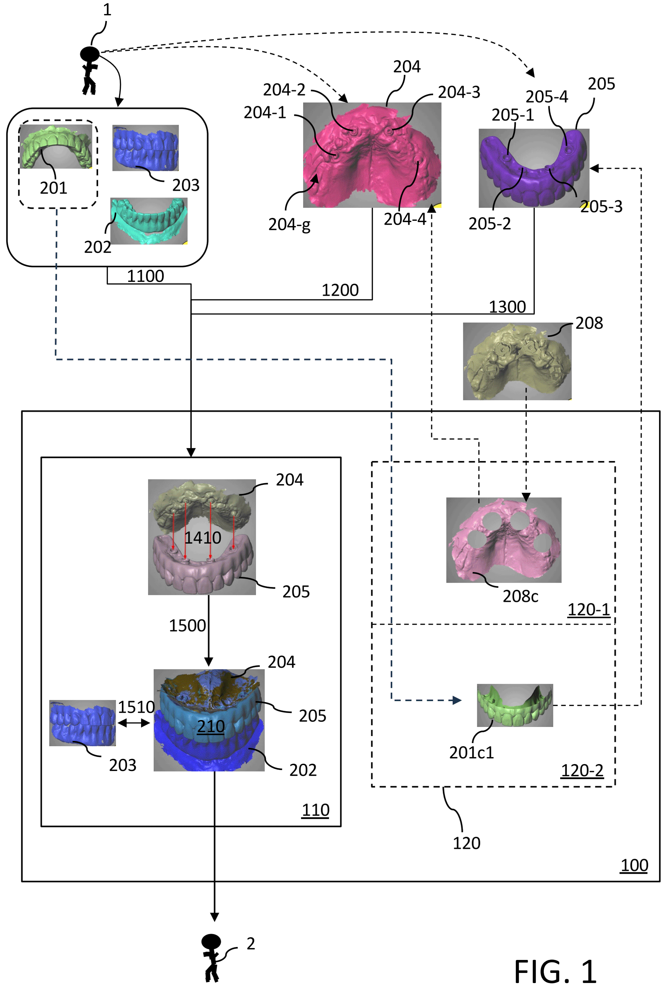

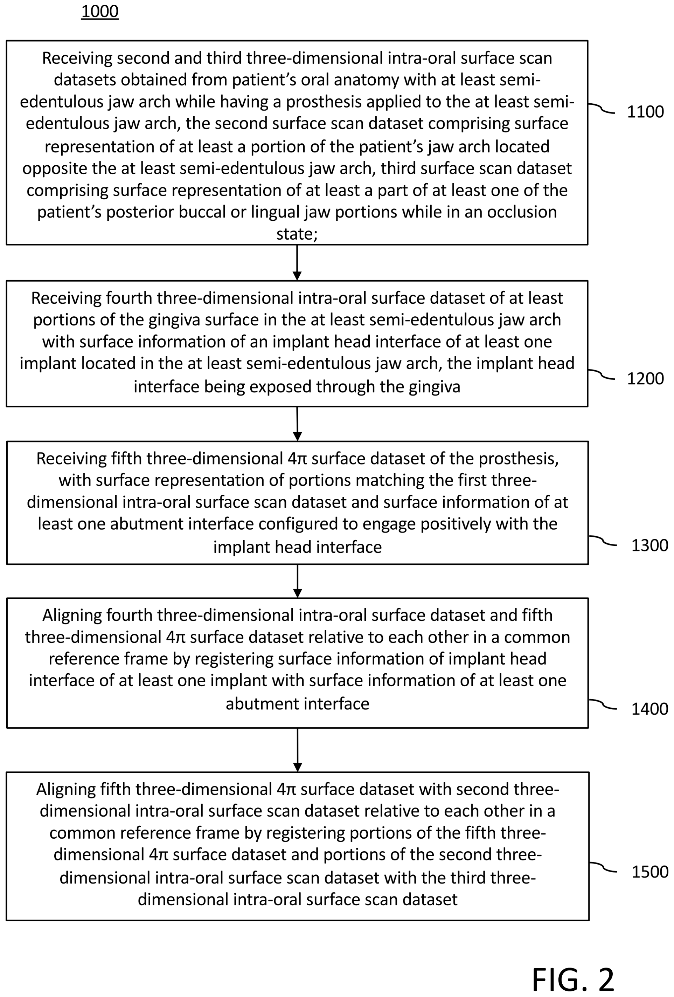

FIG. 1 includes a schematic diagram showing an embodiment of a first aspect of the computer system 100 with respective datasets as inputs to the system for generating a three-dimensional digital patient model 210 of a patient's oral anatomy. System 100 is adapted to execute a computer-implemented method 1000 a depicted by the flowchart of FIG. 2. Therefore, the functions of system 100 will be described in view of method 1000. System 100 has a data interface adapted to receive a plurality of datasets 201 to 205, 208 as inputs.

The input datasets used by a first embodiment are now described in more detail. In this first embodiment, datasets 201 and 208 are optional (illustrated by dashed lines). Datasets 202, 203 are three-dimensional intra-oral surface scan datasets obtained from the oral cavity of a patient 1. Intra-oral scanners for obtaining such surface scan datasets are known in the art and commercially available. The scan datasets 202, 203 received 1100 by system 100 represent a current (medical) state of elements of the patient's oral anatomy obtained in a non-occlusion state and an occlusion state of the patient's oral cavity. The patient has an at least semi-edentulous jaw arch while having a prosthesis applied to the at least semi-edentulous jaw arch.

In the example of FIG. 1, the patient's upper jaw is a fully-edentulous jaw. That is, patient 1 in the example has no teeth in the upper jaw. The patient is wearing a prosthesis applied to the at least semi-edentulous jaw arch while the scan datasets 202 and 203 are obtained. In the example, the prosthesis, as represented by dataset 205, is mounted on the edentulous jaw of the patient via abutment interfaces 205-1 to 205-4 which substantially positively engage with corresponding implant head interfaces of respective implants applied to the patient's upper jaw bone. The Position and orientation of each abutment interface and its respective implant head interface are such that the prosthesis matches the edentulous jaw arch and the patient's bite in the same manner as the patient's original teeth of the upper jaw.

Scan dataset 202 has been obtained from the patient 1 while the oral cavity being in a non-occlusion state, and scan dataset 203 has been obtained from the patient while the oral cavity being in an occlusion state.

The example of FIG. 1 also shows optional first three-dimensional intra-oral surface scan dataset 201 comprising a digital three-dimensional surface representation of the patient's semi-edentulous jaw arch in the upper jaw. The optional scan dataset 201 is not needed for the first embodiment of the first aspect but may be used as a further system input in an optional embodiment of system 100 which is described further down below.

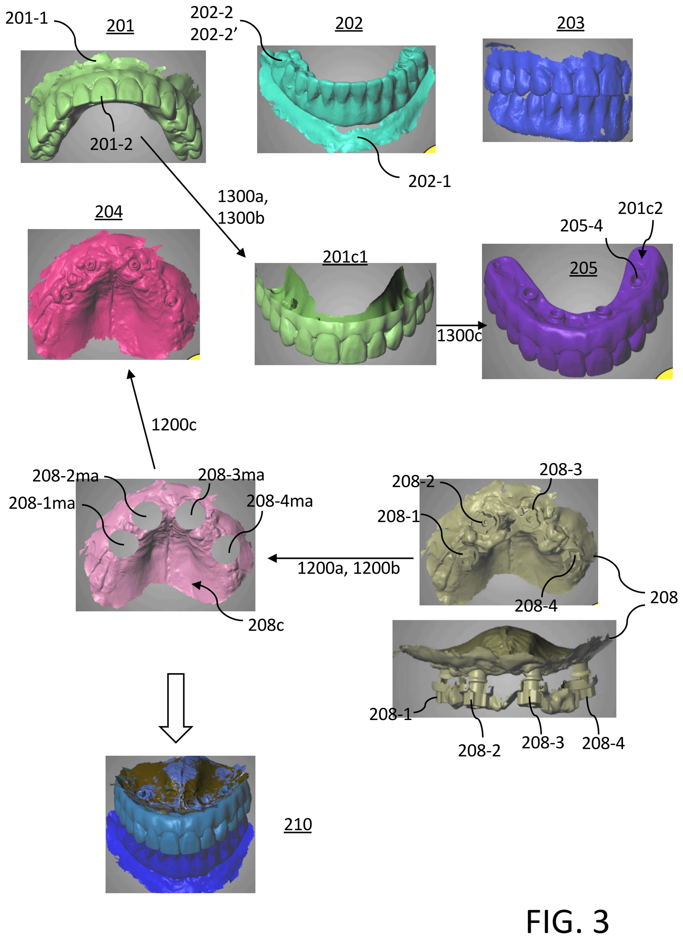

FIG. 3 summarizes surface datasets which are used in the approach for the digital patient model generation, also in optional embodiments. The illustration of optional intra-oral surface scan dataset 201 shows that the scan of the patient's upper fully-edentulous jaw arch includes a representation of the prosthesis 201-2 while being applied to the fully-edentulous jaw. Adjacent to the prosthesis 201-1, the intra-oral scanner has also obtained parts of the patient's soft tissue 201-1 which covers the edentulous jaw. As the bite plane of the prosthesis has been scanned, the scanning was performed with the patient's mouth being opened (non-occlusion state).

The second three-dimensional intra-oral surface scan dataset 202 is received 1100 by the first embodiment of system 100 and comprises a digital three-dimensional surface representation of the patient's jaw arch located opposite the at least semi-edentulous jaw arch. In the example, the second scan data set 202 represents the patient's lower jaw with natural teeth. Turning again briefly to FIG. 3, the second scan dataset 202 includes a representation of the patient's row of teeth 202-2 of the lower jaw. In case the lower jaw of the patient would also be edentulous with a further prosthesis applied to it (second embodiment of system 100), the second scan dataset 202 would include a representation 202-2' of this further prosthesis. In both cases, adjacent to the teeth or to the further prosthesis of the lower jaw, a representation of parts of the patient's soft tissue 202-1 covering the lower jaw is obtained as part of the second scan dataset 202.

In the example, the third three-dimensional intra-oral surface scan dataset 203 comprises a digital three-dimensional surface representation of the patient's upper and lower jaws while being in the occlusion state. Such a scan is also referred to as bite scan. It shows how the patient's upper and lower jaws match in the occlusion state while wearing the prosthesis. In general, it is sufficient for bite registration to obtain scan data from at least a part of at least one of the patient's posterior buccal or lingual jaw portions while in the occlusion state. A full scan of the upper and lower jaws, as shown in the third dataset 203 of the example, is not required for this purpose.

The system 100 further receives 1200 a fourth three-dimensional intra-oral surface dataset 204 of at least portions of the gingiva surface 204-g in said at least semi-edentulous jaw arch of the patient's oral anatomy. In the example, the fourth surface dataset 204 includes the representation of the gingiva surface of the patient's upper jaw also revealing sections which are hidden by the prosthesis when applied. That is, the surface data of the edentulous jaw covered by the gingiva under the prosthesis 205 when applied is included. In the example, the upper edentulous jaw arch also includes implants serving to place the prosthesis in the correct position when mounted thereto. The representations of the respective implant head interfaces 204-1 to 204-4 are exposed through the gingiva and form part of the fourth intra-oral surface dataset 204, too.

It is important for the long-term stability of the prosthesis that the position and orientation of each implant head interface matches the position and orientation of the corresponding abutment interface of the prosthesis to prevent any tension or stress exerted on the patient's soft and or hard tissue such as the gingiva or the jaw bone. Therefore, any digital patient model which serves the design and fabrication of a respective prosthesis must include an accurate position and orientation of each implant head interface to minimize the risk that a prosthesis fabricated with the abutment interfaces positively engaging the respective implant head interfaces, when being mounted on the implant head interfaces, exerts any stress or strain on the respective implants. For this purpose, the fourth surface dataset 204 comprises respective accurate surface information of the implant head interfaces 204-1, 204-2, 204-3, 204-4 in the at least semi-edentulous jaw arch, in particular, the accurate position and/or orientation thereof, which allows to design an adequately matching prosthesis.

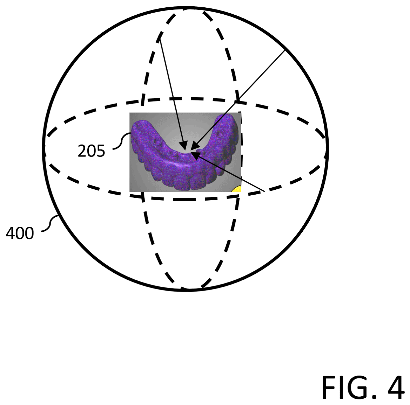

Further, system 100 receives 1300 a fifth three-dimensional 4π surface dataset 205 of the prosthesis. In general, a 4π surface dataset is to be understood as a dataset which includes a representation of a three-dimensional object which can be viewed under substantially all possible viewing angles from the surface of a virtual sphere 400 surrounding said object as illustrated in FIG. 4 for the prosthesis 205. Importantly, it is not necessary, that all surface mesh information is always without gaps or holes when viewed from every possible viewing angle; rather, defects in a surface mesh may be repaired by manual or automatic processes based on statistical information or information from other surface mesh datasets generated in the process of the creation of the digital patient model.

The three arrows in FIG. 4 are exemplary for three different viewing angles. The fifth surface dataset 205 comprises a three-dimensional surface representation of the portions 201-2 of the prosthesis which are visible for an oral scanner when the prosthesis is applied (e.g., the portions matching the optional first intra-oral surface scan dataset 201) as well as surface information of the abutment interfaces 205-1, 205-2, 205-3, 205-4.

FIG. 3 illustrates an example embodiment for one option to generate the fifth surface dataset 205 by reusing an already existing scan dataset. In this optional embodiment, the system also receives the first intra-oral surface scan dataset 201. A sub-module 120-2 of an optional generation module 120 of system 100 generates 1300a a clone copy (e.g., a deep copy) from the first surface scan dataset 201. From this clone copy, the generation module removes 1300b soft tissue representations 201-1 from the clone copy to obtain a first portion 201c1 of the fifth three-dimensional 4π surface dataset 205.

The first portion 201c1 cannot include the surface portion of the prosthesis which comprises the abutment interfaces because during, the scanning of the first surface scan dataset 201, the prosthesis was applied to the edentulous jaw arch of the patient. The prosthesis is then removed from the patient's oral cavity and a continued surface scan of the prosthesis is performed outside the patient's mouth (extra-oral) in that only a complementary second portion 201c2 of the fifth 4π surface dataset 205 may be scanned which includes such prosthesis surface portions occluded in the first surface scan dataset 201.

Once the complementary second portion 201c2 has been obtained 1300c, it may be merged with the first portion 201c1 into the final fifth 4π surface dataset which then includes the surface information of the abutment interfaces 205-1, 205-2, 205-3, 205-4. Other options to obtain the fifth 4π surface dataset 205 with the surface information of at least one abutment interface 205-1, 205-2, 205-3, 205-4 were described already before (performing a full extra oral surface scan of the prosthesis, using an existing digital three-dimensional geometric model of the prosthesis, etc.).

At this stage, system 100 has received all surface datasets which can be relevant to the first aspect of system 100 for generating the digital patient model 210 of the patient's oral anatomy. For precisely matching the reality of the patient's current medical state, the received surface datasets are then aligned so that they reflect this current medical state in the generated digital patient model 210. The surface datasets 202 to 205 are independently obtained by either independent imaging systems capturing 3D image (scan) data relative to the specific 3D coordinate system of the respective imaging system, or by generating or deriving the dataset from a respective data source.

To cross-mount the 3D surface datasets within a single view pane with having its own 3D coordinate system, the 3D surface datasets need to be aligned with each other. This process is often referred to as scan matching or registration. Methods exist for aligning 3D meshes into a single 3D coordinate system. Two common examples of such methods are intensity-based and feature-based image registration or image alignment algorithms. Herein, the reference frame in a target image is stationary, while other datasets are transformed to match to the target. Intensity-based methods compare intensity patterns in images via correlation metrics, while feature-based methods find correspondence between image features such as points, lines, and contours. Other registration methods are known by a person skilled in the art, in particular with regard to surface mesh representations.

For performing the alignment, system 100 includes an alignment module 110 which is adapted to perform the following alignment steps using an appropriate registration/alignment algorithm.

In a first alignment step 1400, the fourth three-dimensional intra-oral surface dataset 204 and the fifth three-dimensional 4π surface dataset 205 of the prosthesis are aligned relative to each other in a common reference frame by registering 1410 the surface information of the implant head interface 204-1 to 204-4 of the at least one implant with the surface information of the at least one abutment interface 205-1 to 205-4. In the example, the first alignment step ensures that the edentulous jaw portion (the patient's upper jaw arch) accurately matches the applied prosthesis in the digital patient model.

In a second alignment step 1500, the mutually registered surface datasets 204, 205 need to be aligned with the opposing jaw arch of the patient while in the occlusion state. In the example, the opposing jaw arch is the patient's lower jaw arch with original teeth represented by the second three-dimensional intra-oral surface scan dataset 202.

The second surface scan dataset 202 is aligned with the mutually registered datasets 204, 205 to generate the final digital patient model 210 in the context of the bite scan dataset 203. For this purpose, the alignment module 110 aligns 1500 the fifth three-dimensional 4π surface dataset 205 of the prosthesis with the second three-dimensional intra-oral surface scan dataset 202 of the lower jaw arch relative to each other in a common reference frame by registering 1510 portions of the fifth 4π surface dataset 205 and portions of the second three-dimensional intra-oral surface scan dataset 202 with the third three-dimensional intra-oral surface scan dataset 203 (bite scan dataset).

The generated digital patient model 210 may then be provided to an operator 2 to be used as a specification for the design and fabrication of a new prosthesis which, for example, can be rigidly attached to the implant heads of the edentulous jaw (e.g., by using screws which match the internal threads of the implants). Alternatively or in addition, the digital patient model 210 may be used for producing, such as, by printing, a physical model of the patient's oral anatomy.

In a second aspect, the disclosed system 100 and method 1000 can be used to also generate a digital patient model in cases where the patient has an at least semi-edentulous jaw arch in both, the upper and the lower jaws. In the example of FIG. 1 and FIG. 3, in addition to the prosthesis 201-2 in the upper jaw, a further prosthesis would be applied to an at least semi-edentulous lower jaw. In the second aspect, in FIG. 3, the representation 202-2 of the real teeth is replaced by a representation 202-2' of the further prosthesis 202-2' in the second surface scan dataset 202.

The second surface scan dataset 202 is, however, optional in the second aspect and not necessary for the generation of the digital patient model. In other words, in the second embodiment, only the third intra-oral scan dataset 203 may be received 1100 by system 100 (the bite scan with two prosthesis applied to the upper and lower edentulous jaw arches).

In this embodiment of the second aspect, the steps which have been previously applied to the surface datasets of upper jaw representations are similarly applied to corresponding surface datasets of the lower jaw representations. In other words, system 100 further receives sixth and seventh three-dimensional surface datasets (not shown). The sixth surface dataset comprises at least portions of the gingiva surface that is located opposite the gingiva surface 204-g of the fourth intra-oral surface dataset 204. That is, the sixth surface dataset corresponds to the fourth dataset for the lower jaw of the patient and comprises surface information of at least a further implant head interface of at least one further implant located in the further at least semi-edentulous jaw arch (the edentulous lower jaw arch in the example). Again, the further implant head interface is exposed through the gingiva (of the lower jaw). The seventh surface dataset (not shown) is a further 4π surface dataset of the further prosthesis (applied to the lower edentulous jaw arch) which is similar to the fifth surface dataset (for the upper jaw arch). The sixth and seventh three-dimensional surface datasets have interface elements (abutment interfaces, implant head interfaces) similar to the fourth and fifth three-dimensional surface datasets.

The alignment module 110 can then align the sixth and seventh three-dimensional surface datasets relative to each other, and to the fourth and fifth three-dimensional surface datasets in the common reference frame by registering the mutually aligned sixth and seventh surface datasets with the third surface dataset 203. As a result of the second embodiment, an accurate digital patient model is generated for a patient having edentulous jaw portions in the upper and lower jaw arches.

In one embodiment, the position and orientation of a particular implant head interface is derived from an eighth three-dimensional intra-oral surface scan dataset 208 obtained from said at least semi-edentulous jaw arch while having the prosthesis removed. This can be performed by a further sub-module 120-1 of the optional generation module 120.

In this embodiment, a scan-body is mounted on the respective implant. In the example of FIG. 3, 4 scan-bodies 208-1, 208-2, 208-3, 208-4 are mounted on respective implant head interfaces exposed through the gingiva of the upper jaw of the patient. This can be done by removing the prosthesis from the patient's oral cavity after the first, second and third surface scans have been obtained. Then, the scan-bodies are affixed to, such as, for example, screwed onto, the respective implants.

FIG. 3 shows the eighth intra-oral surface scan dataset 208 from two different viewing angles. The upper view is a top view on the scan-bodies 208-1, 208-2, 208-3, 208-4 showing the horizontal geometry of the scan-body heads indicating the horizontal positions of the scan-bodies in relation to the patient's gingiva. The lower view is a front view (from the opening of the oral cavity) showing the vertical geometry of the scan-bodies indicating the vertical positions of the scan-bodies (in z-direction) in relation to the patient's gingiva.

As the geometry of the scan-bodies is known, sub-module 120-1 can compute position and orientation of the corresponding implant head interfaces 204-1 to 204-4 (also having a known geometry). The position and orientation of each scan-body can be detected 1200a from the eighth three-dimensional surface scan dataset 208. For deriving the fourth surface dataset 204 from the eighth surface scan dataset, sub-module 120-1 is cleaning 1200b a modification area 208-1ma, 208-2ma, 208-3ma, 208-4ma around each detected scan-body position. Each modification area denotes a part of the eighth three-dimensional surface scan dataset 208 which includes a representation of a respective scan-body which is to be removed.

In the example of FIG. 3, surface dataset 208c illustrates an example where the modification areas are defined as circles with a given radius around the detected scan-body positions. A skilled person may also choose other shapes for the modification areas. The surface within each modification area has a three-dimensional structure. Therefore, cleaning the modification area in this example means to set the values of all surface voxels in the modification area to '0'. In a surface mesh representation, the corresponding action would be removing vertices in the cleaned area so that the surface mesh has a hole.

In the example in FIG. 3, each modification area is computed as a hole being punched by a virtual cylindric tool into the eighth surface scan dataset with the detected scan-body position being at the center of the respective cylinder cross-section. For cleaning 1200b a modification area (removing scan-body representations from the dataset), the skilled person can use alternative known methods such as: generating a clone (e.g., deep) copy from the eighth surface scan dataset 208 with a modification tool (e.g., brush and/or lasso tool) to remove each scan-body representation from the clone copy, or generating a deep copy or a shallow copy from the eighth surface scan dataset 208 and automatically removing, from the clone copy, each scan-body representation by a respectively trained artificial intelligence tool.

For supplementing the cleaned modification areas, a person skilled in the art can select from various alternatives. In one alternative, the scan-bodies are physically removed again from the implant head interfaces after the eighth three-dimensional intra-oral surface scan dataset 208 has been obtained. Then, a three-dimensional surface scan dataset of the patient's gingiva of the at least semi-edentulous jaw arch is obtained for each cleaned modification area. The obtained gingiva surface scan datasets (or mesh representations derived therefrom) can then be merged into the respective cleaned modification areas. The obtained gingiva surface scan datasets associated with the modification areas include the three-dimensional representations of the respective implant head interfaces which are exposed through the gingiva. The computed positions and orientations of the implant head interfaces are taken into account when supplementing the cleaned modification areas so that the implant head interface representations are inserted such that the resulting fourth surface dataset 204 reflects the real intra-oral upper jaw dimensions of the patient in particular with regard to the position and orientation of the implants/implant head interfaces with high accuracy.

In another alternative, the supplementing 1200c of the cleaned modification areas with the surface information of the respective implant head interfaces is computed in accordance with the detected position and orientation of each respective scan-body and the known geometries of the scan-bodies and the implants/implant head interfaces.

In a practical application, a dental specialist can apply the herein disclosed methods in daily practice, for example, as described in the following: The dental specialist initially uses an intra oral scan device to obtain three-dimensional intra-oral surface scan datasets from the patient's oral anatomy. Thereby, the scan device is introduced into the patient's oral cavity and respective scans of the oral anatomy are preformed as required by the first or second embodiment.

In the application of the first aspect, at least the second and third intra oral surface scan datasets are obtained. For the second embodiment, the third intra oral surface scan dataset is readily sufficient. In cases where the 4π surface datasets of a respective prosthesis are partially derived from intra oral surface scan datasets obtained with the prosthesis applied, the dental specialist may also obtain the first three-dimensional intra-oral surface scan datasets while the scan device is inside the patient's oral cavity.

In embodiments which use scan-bodies to obtain the fourth three-dimensional intra-oral surface dataset, the dental specialist would then remove the scan device and the respective prosthesis from the patient's oral cavity. Then, scan-bodies are mounted to the implant head interfaces which are exposed through the gingiva of the semi-edentulous jaw portions of the patient. With the scan-bodies applied, the dental specialist obtains a further three-dimensional intra oral surface scan dataset (scan-body scan) which allows the computer system to compute the positions and orientations of the respective implant head interfaces with high accuracy.

In an embodiment where the fourth three-dimensional intra-oral surface dataset is derived from the scan-body scan by supplementing cleaned modification areas with surface information of a respective gingiva surface scan data set, the dental specialist would first remove the scan-bodies again from the implant head interfaces and then use the scan device again to obtain a gingiva surface scan dataset of the edentulous jaw portions of the patient which is then used by the computer system to supplement the cleaned modification areas of the scan-body scan with respective surface information of the gingiva surface scan dataset.

For obtaining a three-dimensional 4π surface dataset comprising three-dimensional surface representation of the respective prosthesis, the dental specialist can preload in the computer system a copy of a corresponding intra oral scan dataset which has been already obtained of a respective at least semi-edentulous jaw arch with the prosthesis applied, and, after soft tissue representation have been removed from this copy, continue an external surface scan (outside the patient's oral cavity) of the surface portions of said prosthesis which were hidden in the original surface scan dataset of the at least semi-edentulous jaw arch.

Alternatively, the dental specialist can create a full external surface scan of the entire prosthesis or even retrieve an already existing three-dimensional model (e.g., a CAD model) of the prosthesis from a respective data source and provide it as input to the computer system for the alignment steps.

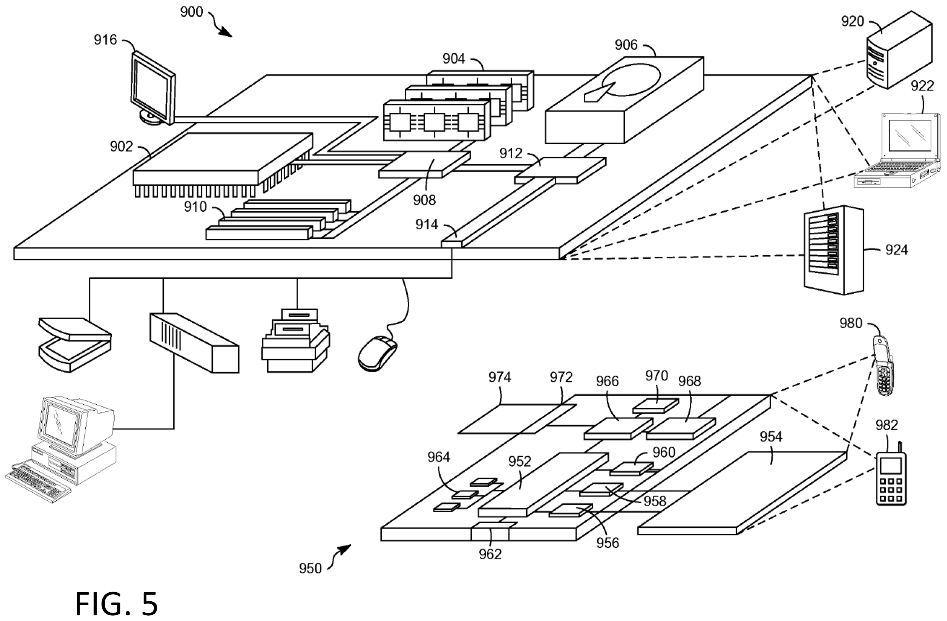

FIG. 5 is a diagram that shows an example of a generic computer device 900 and a generic mobile computer device 950, which may be used with the techniques described here. Computing device 900 is intended to represent various forms of digital computers, such as laptops, desktops, workstations, personal digital assistants, servers, blade servers, mainframes, and other appropriate computers. Generic computer device 900 may correspond to the computer system 100 of FIG. 1. Computing device 950 is intended to represent various forms of mobile devices, such as personal digital assistants, cellular telephones, smart phones, and other similar computing devices. For example, computing device 950 may be used as a GUI frontend for a user to visualize the generated digital patient model 210 of FIG. 1 for the user 2. The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low-speed interface 912 connecting to low-speed bus 914 and storage device 906. The processor 902, the memory 904, the storage device 906, the high-speed interface 908, the high-speed expansion ports 910 and the low-speed interface 912 are interconnected using various busses, and may be mounted on a common motherboard or in other manners as appropriate.

The processor 902 can process instructions for execution within the computing device 900, including instructions stored in the memory 904 or on the storage device 906 to display graphical information for a GUI on an external input/output device, such as display 916 coupled to high-speed interface 908. In other implementations, multiple processing units and/or multiple buses may be used, as appropriate, along with multiple memories and types of memory. Also, multiple computing devices 900 may be connected, with each device providing portions of the necessary operations (e.g., as a server bank, a group of blade servers, or a processing device).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid state memory device, or an array of devices, including devices in a storage area network or other configurations.

A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high-speed interface 908 manages bandwidth-intensive operations for the computing device 900, while the low-speed interface 912 manages lower bandwidth-intensive operations. Such allocation of functions is exemplary only. In one implementation, the high-speed interface 908 is coupled to memory 904, display 916 (e.g., through a graphics processor or accelerator), and to high-speed expansion ports 910, which may accept various expansion cards (not shown). In the implementation, low-speed interface 912 is coupled to storage device 906 and low-speed expansion bus 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as computing device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The computing device 950 may also be provided with a storage device, such as a microdrive or other device, to provide additional storage. The processor 952, the memory 964, the display 954, the communication interface 966, and the transceiver 968 are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processing units. The processor may provide, for example, for coordination of the other components of the computing device 950, such as control of user interfaces, applications run by computing device 950, and wireless communication by computing device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provide in communication with processor 952, so as to enable near area communication of computing device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to computing device 950 through expansion interface 982, which may include, for example, a SIMM (Single In Line Memory Module) card interface. Such expansion memory may provide extra storage space for computing device 950, or may also store applications or other information for computing device 950. Specifically, expansion memory may include instructions to carry out or supplement the processes described above, and may include secure information also. Thus, for example, expansion memory may act as a security module for computing device 950, and may be programmed with instructions that permit secure use of computing device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory, or memory on processor 952, that may be received, for example, over transceiver 968 or external interface 962.

Computing device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through transceiver 968. In addition, short-range communication may occur, such as using a Bluetooth, WiFi, or other such transceiver (not shown). In addition, GPS (Global Positioning System) receiver module may provide additional navigation- and location-related wireless data to computing device 950, which may be used as appropriate by applications running on computing device 950.

Device 950 may also communicate audibly using audio codec 960, which may receive spoken information from a user and convert it to usable digital information. Audio codec 960 may likewise generate audible sound for a user, such as through a speaker, e.g., in a handset of computing device 950. Such sound may include sound from voice telephone calls, may include recorded sound (e.g., voice messages, music files, etc.) and may also include sound generated by applications operating on computing device 950.

The computing device 950 may be implemented in a number of different forms, as shown in the figure. For example, it may be implemented as a cellular telephone 980. It may also be implemented as part of a smart phone, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network ("LAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

A number of embodiments have been described. Nevertheless, it will be understood that various modifications may be made without departing from the spirit and scope of the invention. Particularly, these embodiments comprise features that are interchangeable and could also be combined with other embodiments, not described in detail, however, within the scope of the claims or the general description.

The logic flows depicted in the figures do not require the particular order shown, or sequential order, to achieve desirable results. In addition, other steps may be provided, or steps may be eliminated, from the described flows, and other components may be added to, or removed from, the described systems. Accordingly, other embodiments are within the scope of the following claims.

## Claims

1. A computer-implemented method (1000) for generating a three-dimensional digital patient model (210) of a patient's oral anatomy with the patient (1) having a first at least semi-edentulous jaw arch, and having at least one first dental implant placed in the jawbone of said first at least semi-edentulous jaw arch, the three-dimensional digital patient model providing an accurate representation of the position of said first implant in the patient's jawbone as well as the first at least semi-edentulous jaw arch relative to the opposing jaw of the patient, the method comprising:
Receiving (1100) second and third three-dimensional intra-oral surface scan datasets obtained from the patient's oral anatomy including the first at least semi-edentulous jaw arch while having a first prosthesis applied thereto, wherein
the second three-dimensional intra-oral surface scan dataset (202) comprises a digital three-dimensional surface representation of at least a portion of the patient's jaw arch located opposite the first at least semi-edentulous jaw arch, and
the third three-dimensional intra-oral surface scan dataset (203) comprises a digital three-dimensional surface representation of at least a part of at least one of the patient's posterior buccal or lingual jaw portions while in an occlusion state;
Receiving (1200) a fourth three-dimensional intra-oral surface dataset (204) of at least portions of the gingiva surface (204-g) of said first at least semi-edentulous jaw arch of the patient's oral anatomy, wherein the fourth three-dimensional intra-oral surface dataset comprises surface information of a first implant head interface (204-1, 204-2, 204-3, 204-4) of the at least one first implant located in the first at least semi-edentulous jaw arch, the first implant head interface being exposed through the gingiva;
Receiving (1300) a fifth three-dimensional 4π surface dataset (205) comprising a three-dimensional surface representation of the first prosthesis, as well as surface information of at least one first abutment interface (205-1, 205-2, 205-3, 205-4) configured to engage positively with the first implant head interface (204-1, 204-2, 204-3, 204-4) of the at least one first implant located in the first at least semi-edentulous jaw arch when the first prosthesis is applied;
Aligning (1400) the fourth three-dimensional intra-oral surface dataset (204) and the fifth three-dimensional 4π surface dataset (205) of the first prosthesis relative to each other in a common reference frame by registering (1410) the surface information of the first implant head interface of the at least one first implant with the surface information of the at least one first abutment interface, and
Aligning (1500) the fifth three-dimensional 4π surface dataset (205) with the second three-dimensional intra-oral surface scan dataset (202) relative to each other in the common reference frame by registering portions of the fifth three-dimensional 4π surface dataset (205) and portions of the second three-dimensional intra-oral surface scan dataset (202) with portions of the third three-dimensional intra-oral surface scan dataset (203).

2. A computer-implemented method (1000) for generating a three-dimensional digital patient model (210) of a patient's oral anatomy with the patient (1) having a first at least semi-edentulous jaw arch and a second at least semi-edentulous jaw arch, and having at least one first dental implant placed in the jawbone of said first at least semi-edentulous jaw arch and at least one second dental implant placed in the jawbone of said second semi-edentulous jaw arch, the three-dimensional digital patient model providing an accurate representation of the position of said first and second implants in the patient's jawbones as well as the first and second at least semi-edentulous jaw arch relative to one another, the method comprising:
Receiving (1100) a third three-dimensional intra-oral surface scan dataset obtained from the patient's oral anatomy including the first at least semi-edentulous jaw arch while having a first prosthesis applied to the first at least semi-edentulous jaw arch and a second prosthesis applied to the second at least semi-edentulous jaw arch, wherein the third three-dimensional intra-oral surface scan dataset (203) comprises a digital three-dimensional surface representation of at least a part of the patient's posterior buccal or lingual jaw portions while in an occlusion state;
Receiving (1200) a fourth three-dimensional intra-oral surface dataset (204) of at least portions of the gingiva surface (204-g) of said first at least semi-edentulous jaw arch of the patient's oral anatomy, wherein the fourth three-dimensional intra-oral surface dataset comprises surface information of a first implant head interface (204-1, 204-2, 204-3, 204-4) of the at least one first implant located in the first at least semi-edentulous jaw arch, the first implant head interface being exposed through the gingiva;
Receiving (1300) a fifth three-dimensional 4π surface dataset (205) comprising a three-dimensional surface representation of the first prosthesis as well as surface information of at least one first abutment interface (205-1, 205-2, 205-3, 205-4) configured to engage positively with the first implant head interface (204-1, 204-2, 204-3, 204-4) of the at least one first implant located in the first at least semi-edentulous jaw arch when the first prosthesis is applied;
Receiving a sixth three-dimensional intra-oral surface dataset of at least portions of the gingiva surface of said second at least semi-edentulous jaw arch of the patient's oral anatomy, wherein the sixth three-dimensional intra-oral surface dataset comprises surface information of a second implant head interface of the at least one second implant located in the second at least semi-edentulous jaw arch, the second implant head interface being exposed through the gingiva;
Receiving a seventh three-dimensional 4π surface dataset comprising a three-dimensional surface representation of the second prosthesis as well as surface information of at least one second abutment interface configured to engage positively with the second implant head interface of the at least one second implant located in the second at least semi-edentulous jaw arch when the second prosthesis is applied; and
Aligning (1400) the fourth three-dimensional intra-oral surface dataset (204) and the fifth three-dimensional 4π surface dataset (205) of the first prosthesis relative to each other in a common reference frame by registering (1410) the surface information of the first implant head interface of the at least one first implant with the surface information of the at least one first abutment interface,
Aligning the sixth three-dimensional intra-oral surface dataset and the seventh three-dimensional 4π surface dataset of the second prosthesis relative to each other in a common reference frame by registering the surface information of the second implant head interface of the at least one second implant with the surface information of the at least one second abutment interface, and
Aligning the fifth three-dimensional 4π surface dataset with the seventh three-dimensional 4π surface scan dataset relative to each other in the common reference frame by registering portions of the fifth three-dimensional 4π surface dataset and portions of the seventh three-dimensional 4π surface scan dataset with respective portions of the third three-dimensional intra-oral surface scan dataset.

3. The method of claim 1 or 2, wherein the position and orientation of the at least one first implant head interface is determined from an eighth three-dimensional intra-oral surface scan dataset (208) obtained from said first at least semi-edentulous jaw arch while having the first prosthesis removed and a first scan-body (208-1, 208-2, 208-3, 208-4) having a known geometry mounted to the first implant,
wherein the position and orientation of the first implant head interface is computed based on the known geometry of the first scan-body as well as its position and orientation, and a known geometry of the first implant head interface of the first implant.

4. The method of claim 2, wherein the position and orientation of the at least one second implant head interface is determined from a nineth three-dimensional intra-oral surface scan dataset obtained from said second at least semi-edentulous jaw arch while having the second prosthesis removed and a second scan-body having a known geometry mounted to the second implant,
wherein the position and orientation of the second implant head interface is computed based on the known geometry of the second scan-body as well as its position and orientation, and a known geometry of the second implant head interface of the second implant.

5. The method of any one of claims 3 or 4, wherein the fourth three-dimensional intra-oral surface dataset (204) and/or the sixth three-dimensional intra-oral surface dataset is derived from the eighth (208) and/or nineth three-dimensional intra-oral surface scan dataset, respectively, by:
Detecting (1200a) the position of at least one first and/or second scan-body, cleaning (1200b), from the eighth and/or nineth three-dimensional intra-oral surface scan dataset, respectively, at least one modification area (208-1ma, 208-2ma, 208-3ma, 208-4ma) around the detected position of the at least one first and/or second scan-body; and
Supplementing (1200c) the at least one cleaned modification area with the surface information of the known geometry of the first and/or second implant head interface of the first and/or second implant having the computed position and orientation of the eighth and/or nineth three-dimensional intra-oral surface scan dataset.

6. The method of claim 5, wherein cleaning (1200c) the at least one modification area in the eighth and/or nineth three-dimensional intra-oral surface scan dataset is performed by any one of:
Generating a deep copy (208c) of the eighth (208) and/or nineth three-dimensional intra-oral surface scan dataset with a data modification tool adapted to remove each scan-body representation from the clone copy (208c), or
Generating a deep copy or a shallow copy (208c) of the eighth (208) and/or nineth three-dimensional intra-oral surface scan dataset and automatically removing, from the copy (208c), each representation of a scan-body by a suitably trained artificial intelligence tool.

7. The method of claim 5 or 6, wherein supplementing the at least one cleaned modification area in the eighth and/or nineth three-dimensional intra-oral surface scan dataset is performed by any one of:
With the respective at least one first and/or second scan-body being physically removed from the respective first and/or second implants, obtaining a three-dimensional intra oral gingiva surface scan dataset for the respective cleaned modification area and supplementing the cleaned scan-body area with a portion of the gingiva surface scan dataset comprising a three-dimensional representation of the respective at least one first and/or second implant head interface, or
Computing, for each cleaned modification area, three-dimensional surface information of a respective implant head interface based on geometry, position and orientation of the respective at least one first and/or second scan-body.

8. The method of any one of the previous claims, wherein receiving (1100) the third three-dimensional intra-oral surface scan dataset (203) further comprises receiving a first three-dimensional intra-oral surface scan dataset (201) and/or receiving a second three-dimensional intra-oral surface scan dataset, obtained from the patient's oral anatomy, wherein
the first three-dimensional intra-oral surface scan dataset (201) comprises a digital three-dimensional surface representation of at least a portion of the patient's first at least semi-edentulous jaw arch with the first prosthesis applied, and
the second three-dimensional intra-oral surface scan dataset (202) comprises a digital three-dimensional surface representation of at least a portion of the patient's second at least semi-edentulous jaw arch with the second prosthesis applied,
and wherein receiving (1300) the fifth and/or seventh three-dimensional 4π surface dataset (205) of the prosthesis further comprises:
Generating (1300a) a clone copy of the first and/or second three-dimensional intra-oral surface scan dataset, respectively;
Removing (1300b) soft tissue representations (201-1) from the clone copy to obtain a first portion (201c1) of the fifth and/or seventh three-dimensional 4π surface dataset (205); and
Obtaining (1300c), from a continued surface scan of the first and/or second prosthesis while removed from the oral anatomy of the patient (1), at least one complementary second portion (201c2) of the fifth and/or seventh three-dimensional 4π surface dataset (205), respectively, comprising portions of the first or second prosthesis, respectively, being hidden in the first and/or second three-dimensional intra-oral surface scan datasets (201), wherein the complementary second portion (205) comprises the surface information of at least one first and/or second abutment interface (205-1, 205-2, 205-3, 205-4), respectively;
or
Obtaining the fifth and/or seventh three-dimensional 4π surface dataset (205) comprising the surface information of at least one first and/or second abutment interface (205-1, 205-2, 205-3, 205-4) from a full 4π surface scan of the first and/or second prosthesis while removed from the oral anatomy of the patient;
or
Obtaining the fifth and/or seventh three-dimensional 4π surface dataset (205) with the surface information of at least one first and/or second abutment interface (205-1, 205-2, 205-3, 205-4) from an existing digital three-dimensional geometric model of the prosthesis.

9. A computer program product for generating a three-dimensional digital patient model (210) of a patient's oral anatomy with the patient (1) having a first at least semi-edentulous jaw arch, and having at least one first dental implant placed in the jawbone of said first at least semi-edentulous jaw arch, comprising computer-readable instructions that, when loaded into a memory of a computing device and processed by one or more processors or the computing device, cause the computing device to perform any of the steps of the computer-implemented method according to any one of claims 1 to 8.

10. A computer system for generating a three-dimensional digital patient model (210) of a patient's oral anatomy with the patient (1) having a first at least semi-edentulous jaw arch, and having at least one first dental implant placed in the jawbone of said first at least semi-edentulous jaw arch, the computer system having one or more functional modules adapted to perform, at runtime of the system, the computer-implemented method according to any one of claims 1 to 8.
